# EUROPEAN PATENT APPLICATION

(11) **EP 4 541 297 A1**
(43) Date of publication of application: **23.04.2025**
(21) Application number: 24206370.9
(22) Date of filing: 14.10.2024
(51) Int. Cl.: A61B 17/16, A61B 17/32

(54) **SURGICAL BUR WITH CONTROLLED POOLING**

(30) Priority: 17.10.2023 US 202363590915 P; 13.08.2024 US 202418802047
(71) Applicant: Medtronic Xomed, LLC, Jacksonville, FL 32216 (US)
(72) Inventor: NGUYEN, Thoai, Jacksonville, FL (US); NEZIRAJ, Juliana, Jacksonville, FL (US)
(74) Representative: Viering, Jentschura & Partner mbB Patent- und Rechtsanwälte

(57) **Abstract**

A surgical instrument includes an outer tubular sleeve configured to receive a middle tubular member coaxially therethrough to define an irrigation pathway therebetween and therealong. An inner tubular shaft is rotatably received within the middle tubular member, a distal end of the inner tubular shaft forming a surgical bur configured to cut tissue or bone upon rotation thereof. One or more fluid exit ports is defined at the end of the irrigation passageway between the distal end of the outer tubular sleeve and the distal end of the middle tubular member. The fluid port(s) is configured to direct irrigation fluid in a narrow stream toward the surgical bur. The annular space between each respective fluid exit port at the end of the irrigation passageway is sealed to prevent irrigation fluid from leaking and obscuring the surgeon's view of the surgical bur.

## Description

### CROSS-REFERENCE TO RELATED APPLICATION

This application claims the benefit of and priority to U.S. Provisional Patent Application No. 63/590,915 filed October 17, 2023, the entire disclosure of which is incorporated by reference herein.

### FIELD

The present disclosure relates to surgical systems for bone cutting or bone shaping, and, more particularly, to surgical burs for cutting and shaping bone.

### BACKGROUND

A surgical instrument for cutting or shaping bone typically includes a motor housing with a grip, a surgical attachment, and a surgical tool. The surgical attachment and the surgical tool may be interchangeable with other surgical attachments and surgical tools. The surgical attachment connects to the motor housing and engages with a motor disposed therein configured to rotate, oscillate or otherwise move the surgical tool upon activation thereof. The surgical tool typically includes an elongated shaft and a surgical cutting head or bur. The elongated shaft extends from the surgical bur, engages with the surgical attachment and is axially rotated, oscillated or otherwise moved upon actuation of the motor.

Surgical burs are used to dissect, cut and/or shape bone during a surgical procedure. Various characteristics of surgical instruments or surgical burs are often designed for particular purposes to improve surgical performance. Such characteristics include cutting efficiency, stability, working length, and visibility. Improving one of these characteristics often negatively affects one or more of the other characteristics. For example, designing a surgical bur with an aggressive cutting flute may improve cutting efficiency, but at the same time, the same cutting flute may impede another aspect of the surgical procedure, e.g., reduces visibility of the surgical site. As another example, when an enhancement is provided in an axial region (or at a tip of the surgical bur) or in a radial region (or at an equator of a surgical bur), cutting performance in other regions of the surgical bur can be negatively affected.

It is common for rotary powered surgical tissue cutting instruments to include irrigation systems for supplying irrigation fluid to the internal operative site. In curved rotary powered surgical tissue cutting instruments, the irrigation fluid is often supplied through a separate irrigation tube that is external to the tubular outer sleeve and that extends alongside the external or outer diameter surface of the tubular outer sleeve. Irrigation fluid is supplied to the irrigation tube through a proximal inlet and is discharged from the irrigation tube through a distal outlet disposed near the distal end of the outer sleeve for discharge of the irrigation fluid at the internal operative site.

Other surgical cutting systems utilize an internally disposed irrigation system whereby fluid passes between the rotating inner member or shaft and the outer sleeve. A metered flow of irrigation fluid is achieved by a sealing annulus at the distal end between the rotary shaft and the outer sleeve which also may include one or more grooves to allow fluid flow from the annular space around the rotary shaft onto the surgical site.

As mentioned above, an enhancement to a characteristic of the surgical bur or surgical instrument may negatively affect another characteristic of the instrument. In the case of an internal irrigation system, the irrigation fluid is dispersed to the surgical site around the rotating shaft. With lower spinning shafts (lower RPM surgical burs), the dispersement pattern is nominal and minimally affects the visibility of the cutting head of the surgical bur during use. However, when higher RPM surgical burs are designed for particular surgical purposes and utilized with the surgical system, the irrigation dispersement pattern tends to negatively affect the surgeon's ability to view the cutting head and surgical field. As a result thereof, an internal irrigation system that can be utilized with higher RPM surgical systems and surgical burs without compromising the visual integrity of the surgical site would be a welcome advancement in the field.

### SUMMARY

Provided in accordance with aspects of the present disclosure is a surgical instrument including an outer tubular sleeve configured to receive a middle tubular member coaxially therethrough to define an irrigation pathway therebetween and therealong to a distal end of the outer tubular sleeve and a distal end of the middle tubular member. The outer tubular sleeve is adapted to connect a source of irrigation fluid to the irrigation pathway. An inner shaft is rotatably received within the middle tubular member, a distal end of the inner shaft forming a surgical bur configured to cut tissue upon rotation thereof. The surgical bur is configured to extend distally beyond the distal end of the outer tubular sleeve. One or more fluid exit ports are defined at the distal end of the irrigation passageway by sealing a portion of the irrigation passageway, the one or more fluid exit ports configured to direct irrigation fluid in a narrow stream toward the surgical bur.

In aspects according to the present disclosure, the middle tubular member includes one or more channels defined within an outer peripheral surface thereof and configured to carry irrigation fluid from the fluid source to a respective fluid exit port. In other aspects according to the present disclosure, the annular space between the distal end of the outer tubular sleeve and the distal end of the middle tubular member is sealed between adjacent fluid exit ports to prevent leakage of the irrigation fluid.

In aspects according to the present disclosure, one fluid exit port is defined between the distal end of the outer tubular sleeve and the distal end of the middle tubular member, wherein the remaining annular space between the distal end of the outer tubular sleeve and the distal end of the middle tubular member is sealed to prevent irrigation fluid from leaking from the annular space and obscuring the surgeon's view of the surgical bur.

In aspects according to the present disclosure, the inner shaft includes a proximal section and a distal section, one or both of the proximal or distal sections being made from a material enabling the respective section to bend allowing the inner shaft to bend in accordance with the shape of the outer tubular sleeve.

In aspects according to the present disclosure, the inner shaft includes a proximal section and a distal section, at least one of the proximal or distal sections including a geometry enabling the respective section to bend allowing the inner shaft to bend in accordance with the shape of the outer tubular sleeve. In other aspects according to the present disclosure, the geometry of the inner shaft allowing it to bend includes segmenting. In still other aspects according to the present disclosure, the inner shaft is segmented via at least one of laser cutting, die cutting, plasma cutting, flame cutting, drilling, metal turning, metal shape cutting, or water jet cutting.

Provided in accordance with aspects of the present disclosure is a surgical instrument including an outer tubular sleeve configured to receive a middle tubular member coaxially therethrough to define an irrigation pathway therebetween and therealong to a distal end of the outer tubular sleeve and a distal end of the middle tubular member. The outer tubular sleeve is adapted to connect a source of irrigation fluid to the irrigation pathway. An inner shaft is rotatably received within the middle tubular member, a distal end of the inner shaft forming a surgical bur configured to cut tissue or bone upon rotation thereof. The surgical bur is configured to extend distally beyond the distal end of the outer tubular sleeve. One or more fluid exit cutouts is defined in the distal end of the outer tubular sleeve at the end of the irrigation passageway and is configured to direct irrigation fluid in a narrow stream toward the surgical bur. The annular space between each respective fluid exit cutout at the end of the irrigation passageway is sealed to prevent irrigation fluid from obscuring the surgeon's view of the surgical bur.

Provided in accordance with aspects of the present disclosure is a surgical instrument including an outer tubular sleeve configured to receive a middle tubular member coaxially therethrough to define an irrigation pathway therebetween and therealong to a distal end of the outer tubular sleeve and a distal end of the middle tubular member. The outer tubular sleeve is adapted to connect a source of irrigation fluid to the irrigation pathway. An inner shaft is rotatably received within the middle tubular member, a distal end of the inner shaft forming a surgical bur configured to cut tissue or bone upon rotation thereof. The surgical bur is configured to extend distally beyond the distal end of the outer tubular sleeve. One or more fluid exit ports is defined proximate the distal end of the outer tubular sleeve at the end of the irrigation passageway and is configured to direct irrigation fluid in a narrow stream toward the surgical bur. The annular space between each respective fluid exit port at the end of the irrigation passageway is sealed to prevent irrigation fluid from obscuring the surgeon's view of the surgical bur.

In aspects according to the present disclosure, the middle tubular member includes one or more channels defined within an outer peripheral surface thereof configured to carry irrigation fluid from the fluid source to a corresponding number of fluid exit ports defined within the outer tubular sleeve. In other aspects according to the present disclosure, the annular space between the distal end of the outer tubular sleeve and the distal end of the middle tubular member is sealed to prevent leakage of the irrigation fluid.

In aspects according to the present disclosure, one fluid exit port is defined within the outer tubular sleeve and wherein the annular space between the distal end of the outer tubular sleeve and the distal end of the middle tubular member is sealed to prevent irrigation fluid from leaking from the annular space and obscuring the surgeon's view of the surgical bur.

In aspects according to the present disclosure, the inner shaft includes a proximal section and a distal section, one or both of the proximal or distal sections being made from a material enabling the respective section to bend allowing the inner shaft to bend in accordance with the shape of the outer tubular sleeve.

In aspects according to the present disclosure, wherein the inner shaft includes a proximal section and a distal section, one or both of the proximal or distal sections including a geometry enabling the respective section to bend allowing the inner shaft to bend in accordance with the shape of the outer tubular sleeve. In other aspects according to the present disclosure, the geometry of the inner shaft allowing it to bend includes segmenting. In still other aspects according to the present disclosure, the inner shaft is segmented via at least one of laser cutting, die cutting, plasma cutting, flame cutting, drilling, metal turning, metal shape cutting, or water jet cutting.

### BRIEF DESCRIPTION OF THE DRAWINGS

The above and other aspects and features of the present disclosure will become more apparent in view of the following detailed description when taken in conjunction with the accompanying drawings wherein like reference numerals identify similar or identical elements.
FIG. 1A is a perspective view of a surgical instrument having a surgical attachment incorporating a surgical bur in accordance with the present disclosure;
FIG. 1B is an enlarged, perspective view of the surgical instrument of the FIG. 1A with the surgical bur and cutting head shown separated from the surgical attachment;
FIGS. 2A and 2B are greatly-enlarged, distal perspective views of prior art surgical burs having non-isolated irrigation paths disposed between the middle tubular member and the outer tubular sleeve causing uncontrolled irrigation proximate the cutting head of the surgical bur;
FIG. 3A is a greatly-enlarged, distal perspective view of a surgical bur in accordance with one embodiment of the present disclosure having a series of fluid exit ports disposed between the middle tubular member and the outer tubular sleeve allowing controlled, stream-like irrigation proximate the cutting head of the surgical bur;
FIG. 3B is a greatly-enlarged, distal perspective view of a surgical bur in accordance with one embodiment of the present disclosure having a series of narrow isolated irrigation channels disposed between the middle member and the outer sleeve allowing controlled, stream-like irrigation proximate the cutting head of the surgical bur;
FIG. 4A is a greatly-enlarged, distal perspective view of a surgical bur in accordance with another embodiment of the present disclosure having an isolated exit hole defined in the outer tubular sleeve causing controlled, stream-like irrigation proximate the cutting head of the surgical bur;
FIG. 4B is a side view of the surgical bur of FIG. 4A in use showing controlled, stream-like irrigation proximate the cutting head of the surgical bur; and
FIG. 5 is a greatly-enlarged, distal perspective view of a surgical bur in accordance with another embodiment of the present disclosure having an isolated cutout defined in the outer tubular sleeve causing controlled, stream-like irrigation proximate the cutting head of the surgical bur;
FIG. 6 is a schematic illustration of an exemplary robotic surgical system configured for use with the surgical instrument of FIGS 1A and 1B, in accordance with aspects of the present disclosure.

### DETAILED DESCRIPTION

Embodiments of the present disclosure are directed to surgical cutting instruments having a surgical bur at a distal end thereof. Generally, the cutting instrument includes an inner shaft rotatably received within a middle tubular member which is coaxially-disposed within an outer tubular sleeve. Inner shaft is referenced hereinafter as tubular but may be include any cross section depending upon a particular purpose. The inner shaft and the outer tubular sleeve each mechanically couple to a hub to facilitate their rotational relationship and their control by a handpiece that further supports both the inner tubular shaft and the outer tubular sleeve. Rotation of the surgical bur via rotation of inner tubular shaft causes cutting and debriding of the target tissue and/or bone at a treatment site.

The outer tubular sleeve includes an interior passage that acts as an irrigation pathway to supply an irrigation fluid to the treatment site along the middle tubular member adjacent to the surgical bur. The middle tubular member may include an exterior passageway along an outer surface thereof that operates in a similar manner to the interior passage. Because the irrigation pathway is incorporated internally and not provided through an external tube (as in conventional cutting instruments), the surgical cutting instrument has a low cross-sectional profile facilitating introduction of the cutting head through narrow passageways to the treatment site.

In some embodiments, the bur and the inner tubular shaft further define an aspiration pathway through an interior of the bur (and the inner tubular shaft) to avoid the conventional arrangement of an external aspiration tube of the types typically used in conventional instruments. In these embodiments, the inner tubular shaft has a length so that the aspiration pathway may extend continuously through a hub assembly of both the inner tubular shaft and the outer tubular sleeve. Accordingly, with this arrangement, the internally incorporated aspiration pathway further maintains the low cross-sectional profile that is achieved via arranging the irrigation pathway on the outer peripheral surface of the middle tubular member or within a side wall of the outer tubular sleeve, as described above.

In one embodiment, the instrument can be configured to be utilized in an electromagnetic image guided system, wherein the instrument is equipped with at least one tracking device. One exemplary electromagnetic image guided system is disclosed in U.S. Patent Publication No. 8,504,139, entitled "Navigating A Surgical Instrument". In general, the tracking device can include one or more coils that can provide an indication of position and orientation of the device to the image guided system. This indication can be useful during a surgical procedure that utilizes the surgical instrument.

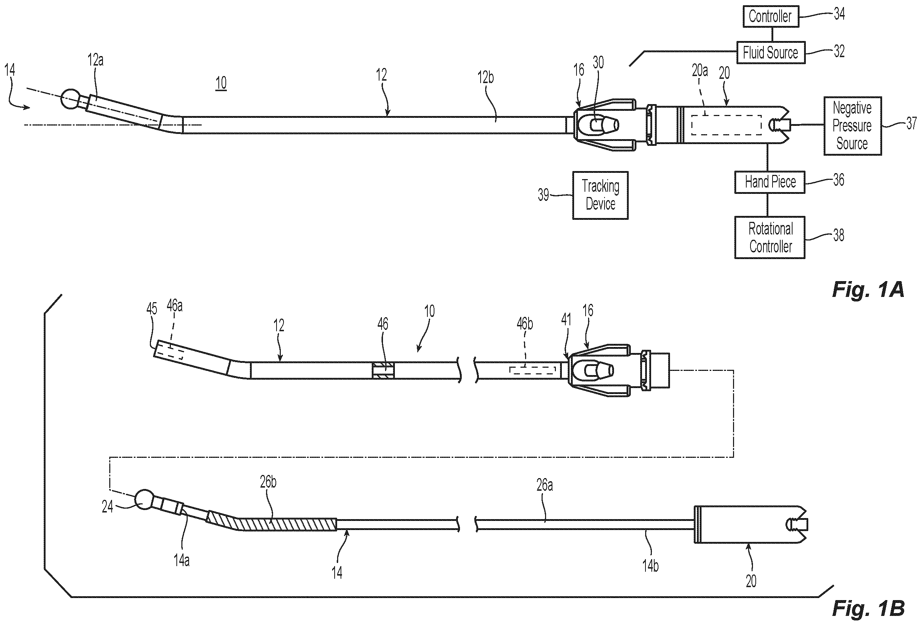

Surgical instruments embodying principles of the present disclosure can be employed in various types of surgery including, but not limited to, various sinus procedures, skull base tumor removal (such as pituitary tumors, clivus chordomas, etc.), mastoidectomy, temporal bone tumor removal, craniotomy, a modified Lothrop procedure, spinal diseases, notchplasty, acromioplasty, laminotomy, laminectomy and the like. These and other embodiments are described more fully in association with FIGS. 1A-5.

One embodiment of a surgical instrument according to the present disclosure, namely, surgical micro-burring instrument 10, is illustrated in FIGS. 1A and 1B. Instrument 10 generally includes an outer tubular sleeve 12, an inner tubular shaft 14, a motor housing 20 and an outer irrigation hub 16. Outer tubular sleeve 12 operably couples to the irrigation hub 16 which, in turn, operably connects to an irrigation source 32. Inner tubular shaft 14 operably couples to the motor housing 20 at a proximal end 14a thereof and is configured to support a surgical bur 24 at a distal end 14b thereof.

The inner tubular shaft 14 is sized to be coaxially-received within a middle tubular member 46, which, in turn, is coaxially-received within the outer tubular sleeve 12. Inner tubular shaft 14 is configured to rotate within the middle tubular member 46 upon actuation of a motor 20a disposed within the motor housing 20. Middle tubular member 46 is coaxially fixed within the outer tubular sleeve 12 upon assembly as explained in more detail below.

The inner tubular member 14 may be sectioned to include a proximal section 26a and a distal section 26b. Either or both the proximal and distal sections 26a, 26b may be made from a material that enables the inner tubular shaft 14 to bend such that the inner tubular shaft 14 can assume a curvature of the outer tubular sleeve 12 depending upon a particular surgical purpose. Various other manufacturing techniques may be employed to enable the inner tubular shaft 14 to bend, e.g., segmenting the respective proximal and/or distal sections 26a, 26b via laser cutting, die cutting, plasma cutting, flame cutting, drilling, metal turning, metal shape cutting, water jet cutting, etc.

As illustrated in FIG. 1A, the outer tubular sleeve 12 extends distally from the irrigation hub 16 which includes one or more irrigation ports 30 configured for fluid communication via tubing (not shown) with the fluid source 32 controlled by a fluid controller 34. During use, the surgical bur 24 generates a high amount of heat as the surgical bur 24 cuts hard tissue and bone. Moreover, the high rotational speeds of the inner tubular shaft 14 within the middle tubular member 46 also generates some degree of heat which is conducted proximally from the surgical bur 24 along the inner tubular shaft 14 and propagates to the outer tubular sleeve 12 over prolonged use.

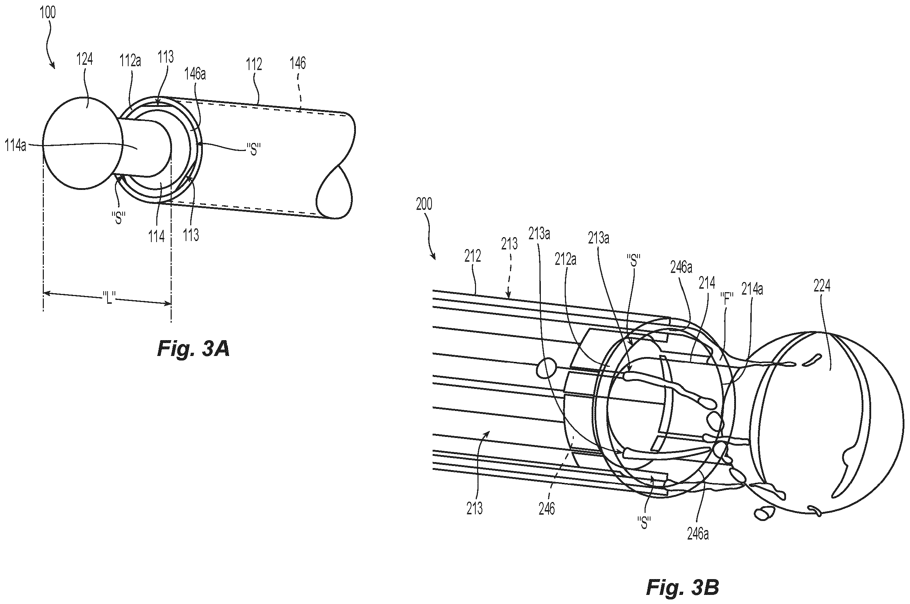

In order to cool the surgical bur 24 and the inner tubular shaft 14, irrigation fluid "F" is fed from the irrigation ports 30 between and along the outer periphery of the middle tubular member 46 and/or the inner periphery of the outer tubular sleeve 12 to irrigate and cool the surgical bur 24 and the outer tubular sleeve 12 during use thereof. One or more channels 46a (see FIG. 3) may be defined within the outer periphery of the middle tubular member 46 or may be defined within the inner periphery (not shown) of the outer tubular sleeve 12 to facilitate transport of the irrigation fluid "F" to a distal end 12a of the outer tubular sleeve 12 to irrigate the surgical bur 24 as explained in more detail below.

As mentioned above, inner tubular shaft 14 operably connects to motor housing 20 which is configured to be engaged by a handpiece 36 for handling instrument 10. In particular, rotational controller 38 (via a connection between handpiece 36 motor housing 20) enables selective rotational control over inner tubular shaft 14 to cause high-speed rotation of bur 24 for debriding or otherwise cutting of target tissue.

Additionally, inner tubular shaft 14 may be coupled to a negative pressure source or vacuum 37 that provides suction to the bur 24 for aspiration purposes. In embodiments, irrigation hub 16 may include or be selectively engageable with a tracking or navigational system 39 such that instrument 10 can be used with various image-guided technologies to track and/or position the surgical bur 24 within a patient's anatomy.

The middle tubular member 46 extends from a proximal end 12b to a distal end 12a of the outer tubular sleeve 12. In this regard, and as described in greater detail below, a distal section of the middle tubular member 46 is open at a distal end 46a thereof to enable the inner tubular shaft 14 to extend distally beyond the distal end 12a of outer tubular sleeve 12. Similarly, a proximal section 46b of the middle tubular member 46 is open at a proximal end 41 thereof to facilitate positioning of the inner tubular shaft 14 within the middle tubular member 46.

As previously described, the inner tubular shaft 14 includes the surgical bur 24 engaged at a distal end 14a thereof. In general terms, bur 24 may be a solid member that can assume a variety of forms and is adapted with an abrasive or rough surface to cut or abrade bodily tissue upon rotation thereof. In some embodiments, the bur 24 forms a cutting surface including one or more cutting elements or flutes. While a spherical bur configuration is shown, it will be appreciated that other configurations can be used including, but not limited to, cylindrical, hemispherical, ellipsoidal, and pear-shaped configurations.

The micro-burring instrument 10 is assembled by coaxially positioning the inner tubular shaft 14 within the outer tubular sleeve 12 via the middle tubular member 46 such that the inner tubular shaft 14 is rotatable relative to the outer tubular sleeve 12 via actuation of the motor 20a. Once assembled, the motor housing 20 abuts against the irrigation hub 16 to a point where the distal end 12a of the outer tubular sleeve 12 is proximal a desired distance to the surgical bur 24.

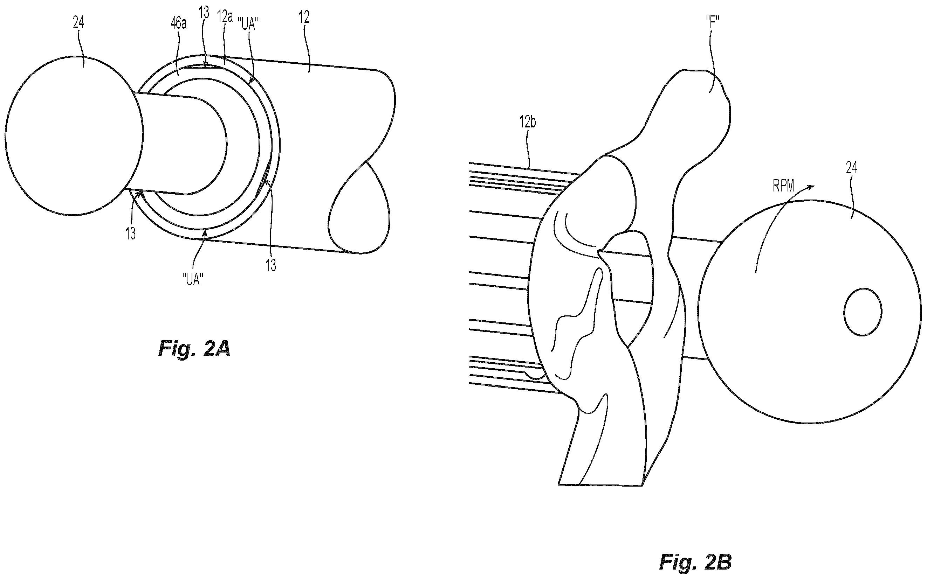

Turning now to FIGS. 2A and 2B, many prior art micro-burring instruments are configured to randomly dispense the irrigation fluid "F" onto the surgical bur 24 as the bur 24 rotates. Under some circumstances with low RPM surgical burs 24, the surgeon's field of view is not compromised, however, with higher RPM surgical burs 24 "RPM^{H}", the fluid "F" becomes more randomly dispersed compromising the surgeon's field of view (see, e.g., FIG. 2B). In some prior art devices, there are no defined ports between the respective distal ends 12a, 46a of the outer tubular sleeve 12 and the middle tubular member 46 and the fluid "F" simply exits through the unsealed annular spaces "UA" therebetween (FIG. 2A). Still in other prior art devices, one or more large fluid channels are disposed along the outer periphery of the middle tubular member 46 to carry fluid "F" therealong to dispense the fluid "F" in a large mass atop the surgical bur 24, again, tending to compromise the surgeon's view with higher RPM surgical burs 24 "RPM^{H}" (See, e.g., FIG. 2B).

FIG. 3A shows one embodiment of a micro-burring instrument 100 according to the present disclosure which includes an inner tubular shaft 114 coaxially and rotationally disposed within a middle tubular member 146 and ultimately connected to a rotational controller 38 and motor 20a (see FIG. 1A). Inner tubular shaft 114 includes a surgical bur 124 disposed at a distal end 114a thereof that is configured to cut tissue or bone upon actuation of the motor 20a.

Middle tubular member 146, in turn, is coaxially-disposed within an outer tubular sleeve 112 and includes a distal end 146a that substantially aligns with the distal end 112a of outer tubular sleeve 112. Surgical bur 124 is configured to extend a distance "L" from the distal ends 146a, 112a which may be determined based on a particular surgical purpose. One or more fluid exit ports 113 may be defined between the respective ends 112a, 146a or the outer tubular sleeve 112 and the middle tubular member 146. Fluid "F" from fluid source 32 may be forced under pressure along the outer periphery of the middle tubular member 146 or within a channel (not shown) defined therealong and out of the fluid exit ports 113 to irrigate the surgical bur 124 during actuation. The annular spaces between the ports 113 are sealed "S" forcing the fluid "F" to only exit at the ports 113. As a result, the fluid "F" exits each port 113 in narrow stream (or fluid is directed in a more narrow fashion) towards the surgical bur 124 regardless of the rotational speed (RPM) of the surgical bur 124 improving the visibility of the surgical field, or put another way, preventing fluid from obscuring the view of the surgical field.

FIG. 3B shows another embodiment of a micro-burring instrument 200 according to the present disclosure which includes an inner tubular shaft 214 coaxially and rotationally disposed within a middle tubular member 246 and ultimately connected to a rotational controller 38 and motor 20a (see FIG. 1A). Inner tubular shaft 214 includes a surgical bur 224 disposed at a distal end 214a thereof that is configured to cut tissue or bone upon actuation of the motor 20a.

Middle tubular member 246, in turn, is coaxially-disposed within an outer tubular sleeve 212 and includes a distal end 246a that substantially aligns with the distal end 212a of outer tubular sleeve 212. Fluid "F" from fluid source 32 may be forced under pressure along the outer periphery of the middle tubular member 246 within a series of narrow channels 213 defined therealong and out of a distal end or port 213a of each channel 213 to irrigate the surgical bur 224 during actuation. The annular space between the ports 213a are sealed "S" forcing the fluid "F" to only exit at the ports 213a. As a result, the fluid "F" exits each port 213a in narrow stream towards the surgical bur 224 regardless of the rotational speed (RPM) of the surgical bur 224 improving the visibility of the surgical field.

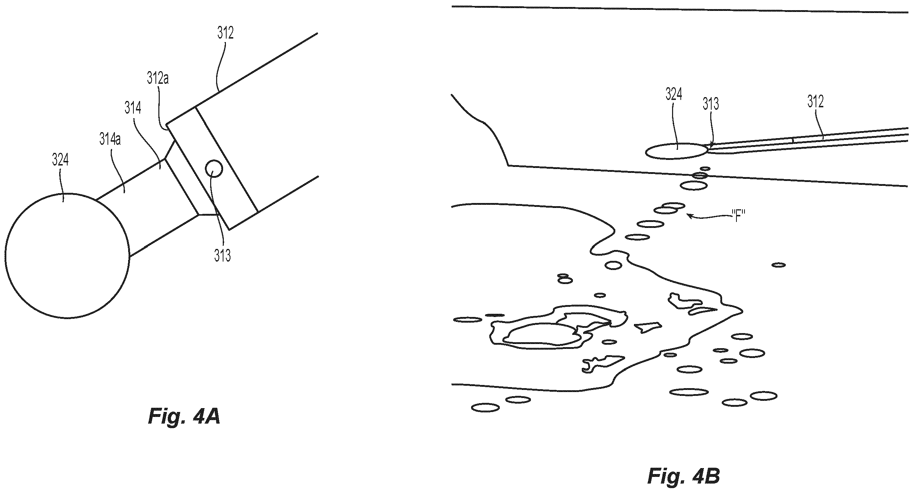

FIGS. 4A and 4B show another embodiment of a micro-burring instrument 300 according to the present disclosure. Similar to the embodiments described above, micro-burring instrument 300 includes an inner tubular shaft 314 coaxially and rotationally disposed within a middle tubular member (not shown) and ultimately connected to a rotational controller 38 and motor 20a (see FIG. 1A). Inner tubular shaft 314 includes a surgical bur 324 disposed at a distal end 314a thereof that is configured to cut tissue or bone upon actuation of the motor 20a.

Middle tubular member, in turn, is coaxially-disposed within an outer tubular sleeve 312 and includes a distal end (not shown) that substantially aligns with the distal end 312a of outer tubular sleeve 312. Fluid "F" from fluid source 32 may be forced under pressure along the outer periphery of the middle tubular member and out of a distal port 313 to irrigate the surgical bur 324 during actuation. The annular space between the distal end of the middle tubular member and the distal end 312a of the outer tubular sleeve 312 is sealed forcing the fluid "F" to only exit at the port 313. As a result, the fluid "F" exits port 313 in narrow stream towards the surgical bur 324 regardless of the rotational speed (RPM) of the surgical bur 324 improving the visibility of the surgical field.

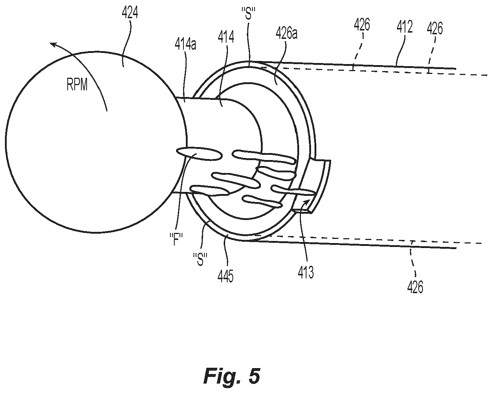

FIG. 5 shows yet another embodiment of a micro-burring instrument 400 according to the present disclosure. Similar to the embodiments described above, micro-burring instrument 400 includes an inner tubular shaft 414 coaxially and rotationally disposed within a middle tubular member 426 and ultimately connected to a rotational controller 38 and motor 20a. Inner tubular shaft 414 includes a surgical bur 424 disposed at a distal end 414a thereof that is configured to cut tissue or bone upon actuation of the motor 20a.

Middle tubular member 426, in turn, is coaxially-disposed within an outer tubular sleeve 412 and includes a distal end 426a that substantially aligns with the distal end 412a of outer tubular sleeve 412. Fluid "F" from fluid source 32 may be forced under pressure along the outer periphery of the middle tubular member 426 and out of a distal cutout 413 to irrigate the surgical bur 424 during actuation. The annular space between the distal end 426a of the middle tubular member 426 and the distal end 412a of the outer tubular sleeve 412 is sealed "S" forcing the fluid "F" to only exit at the cutout 413. As a result, the fluid "F" exits cutout 413 in narrow stream towards the surgical bur 424 regardless of the rotational speed (RPM) of the surgical bur 424 improving the visibility of the surgical field.

Turning briefly back to FIG. 3B, because the irrigation fluid pathway is contained internally within a channel 213 defined with the outer periphery of the middle tubular member, e.g., middle tubular member 246, or, in some cases in a channel (not shown) defined within the outer tubular sleeve 212, the outer tubular sleeve 212 has a smaller overall cross-sectional profile. As a result thereof, this smaller cross-sectional profile provides instrument 200 with greater maneuverability to enable the distal sections of the instrument 200 to pass through various soft tissues and bony structures with less likelihood of the instrument 200 catching on soft tissues and bony structures encountered during initial instrument deployment, instrument orientation and instrument use.

Regardless of exact embodiment of the micro-burring instrument of the present disclosure intended for use in performing various sinus operations and other procedures, the embodiments described herein generally operate in a similar fashion. By way of example, and with reference to the embodiment of FIG. 3A, the assembled instrument 100 is maneuvered to the treatment site and the bur 124 is positioned against the bone or other target tissue. Next, inner tubular shaft 114 is rotated relative to the middle tubular member 126, such that the bur 124 cuts or abrades the contacted target tissue. The bur 124, and thus the target site, are periodically (or continuously) flushed with an irrigation fluid "F" from ports 113.

In addition to the exemplary surgical procedure described above, any of the above-described micro-burring instruments 100, 200, 300, or 400 of the present disclosure can be used to perform a variety of other surgical procedures in which hard tissue is debrided or cut while flooding the treatment site with fluid "F" to irrigate the bur 124 and the target tissue.

As familiar to those skilled in the art, the outer tubular sleeve, e.g., outer tubular sleeve 112 and the inner tubular shaft, e.g., inner tubular shaft 114, may be formed from biocompatible metallic materials, such as stainless steel, titanium alloys, and the like. Accordingly, at least the outer tubular sleeve 112 defines a generally rigid member.

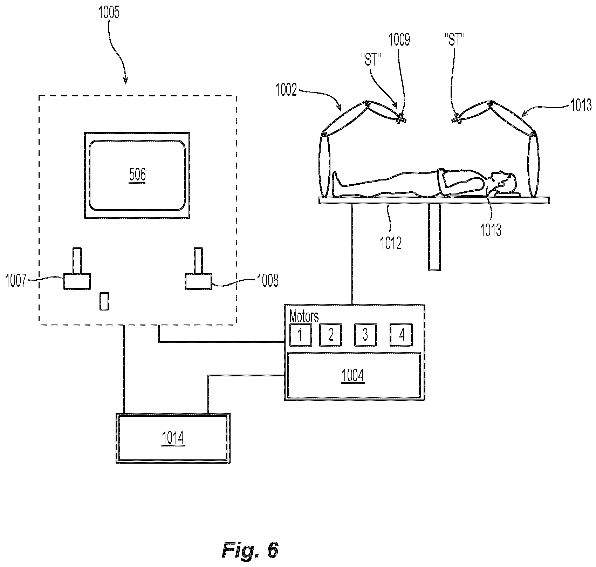

Turning now to FIG. 6, a robotic surgical system 1000 configured for use in accordance with the present disclosure is shown. Aspects and features of robotic surgical system 1000 not germane to the understanding of the present disclosure are omitted to avoid obscuring the aspects and features of the present disclosure in unnecessary detail.

Robotic surgical system 1000 generally includes a plurality of robot arms 1002, 1003; a control device 1004; and an operating console 1005 coupled with control device 1004. Operating console 1005 may include a display device 1006, which may be set up in particular to display three-dimensional images; and manual input devices 1007, 1008, by means of which a clinician, e.g., a clinician, may be able to telemanipulate robot arms 1002, 1003 in a first operating mode. Robotic surgical system 1000 may be configured for use on a patient 1013 lying on a patient table 1012 to be treated in a minimally invasive manner. Robotic surgical system 1000 may further include a database 1014, in particular coupled to control device 1004, in which are stored, for example, pre-operative data from patient 1013 and/or anatomical atlases.

Each of the robot arms 1002, 1003 may include a plurality of members, which are connected through joints, and a mounted device which may be, for example, a surgical tool "ST." The surgical tools "ST" may include, for example, the micro-burring instruments 100 of the present disclosure, thus providing any of the above-detailed functionality on a robotic surgical system 1000.

Robot arms 1002, 1003 may be driven by electric drives, e.g., motors, connected to control device 1004. The motors, for example, may be rotational drive motors configured to provide rotational inputs to accomplish a desired task or tasks. Control device 1004, e.g., a computer, may be configured to activate the motors, in particular by means of a computer program, in such a way that robot arms 1002, 1003, and, thus, their mounted surgical tools "ST" execute a desired movement and/or function according to a corresponding input from manual input devices 1007, 1008, respectively. Control device 1004 may also be configured in such a way that it regulates the movement of robot arms 1002, 1003 and/or of the motors.

Control device 1004, more specifically, may control one or more of the motors based on rotation, e.g., controlling to rotational position using a rotational position encoder (or Hall effect sensors or other suitable rotational position detectors) associated with the motor to determine a degree of rotation output from the motor and, thus, the degree of rotational input provided. Alternatively or additionally, control device 1004 may control one or more of the motors based on torque, current, or in any other suitable manner.

While several aspects of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular aspects. Those skilled in the art will envision other modifications within the scope and spirit of the claims appended hereto.

## Claims

1. A surgical instrument, comprising:
an outer tubular sleeve configured to receive a middle tubular member coaxially therethrough to define an irrigation pathway therebetween and therealong to a distal end of the outer tubular sleeve and a distal end of the middle tubular member, the outer tubular sleeve adapted to connect a source of irrigation fluid to the irrigation pathway;
an inner shaft rotatably received within the middle tubular member, a distal end of the inner shaft forming a surgical bur configured to cut tissue or bone upon rotation thereof, the surgical bur extending distally beyond the distal end of the outer tubular sleeve; and
at least one fluid exit port defined at the end of the irrigation passageway between the distal end of the outer tubular sleeve and the distal end of the middle tubular member configured to direct irrigation fluid in a narrow stream toward the surgical bur, wherein an annular space between each respective fluid exit port at the end of the irrigation passageway is sealed to prevent irrigation fluid from leaking and obscuring the surgeon's view of the surgical bur.

2. The surgical instrument according to claim 1, wherein the middle tubular member includes at least one channel defined within an outer peripheral surface thereof configured to carry irrigation fluid from the fluid source to a corresponding number of fluid exit ports between the distal end of the outer tubular sleeve and the distal end of the middle tubular member.

3. The surgical instrument according to claim 2, wherein the annular space between the distal end of the outer tubular sleeve and the distal end of the middle tubular member is sealed between adjacent fluid exit ports to prevent leakage of the irrigation fluid.

4. The surgical instrument according to any preceding claim, wherein one fluid exit port is defined between the distal end of the outer tubular sleeve and the distal end of the middle tubular member, wherein the remaining annular space between the distal end of the outer tubular sleeve and the distal end of the middle tubular member is sealed to prevent irrigation fluid from leaking from the annular space and obscuring the surgeon's view of the surgical bur.

5. The surgical instrument according to any preceding claim, wherein the inner shaft includes a proximal section and a distal section, at least one of the proximal or distal sections being made from a material enabling the respective section to bend allowing the inner shaft to bend in accordance with the shape of the outer tubular sleeve.

6. The surgical instrument according to any preceding claim, wherein the inner shaft includes a proximal section and a distal section, at least one of the proximal or distal sections including a geometry enabling the respective section to bend allowing the inner shaft to bend in accordance with the shape of the outer tubular sleeve.

7. The surgical instrument according to claim 6, wherein the geometry of the inner shaft allowing it to bend includes segmenting.

8. The surgical instrument according to claims 6 or 7, wherein the inner shaft is segmented via at least one of laser cutting, die cutting, plasma cutting, flame cutting, drilling, metal turning, metal shape cutting, or water jet cutting.

9. A surgical instrument, comprising:
an outer tubular sleeve configured to receive a middle tubular member coaxially therethrough to define an irrigation pathway therebetween and therealong to a distal end of the outer tubular sleeve and a distal end of the middle tubular member, the outer tubular sleeve adapted to connect a source of irrigation fluid to the irrigation pathway;
an inner shaft rotatably received within the middle tubular member, a distal end of the inner shaft forming a surgical bur configured to cut tissue or bone upon rotation thereof, the surgical bur extending distally beyond the distal end of the outer tubular sleeve; and
at least one fluid exit cutout defined in the distal end of the outer tubular sleeve at the end of the irrigation passageway configured to direct irrigation fluid in a narrow stream toward the surgical bur, wherein an annular space between each respective fluid exit cutout at the end of the irrigation passageway is sealed to prevent irrigation fluid from obscuring the surgeon's view of the surgical bur.

10. A surgical instrument, comprising:
an outer tubular sleeve configured to receive a middle tubular member coaxially therethrough to define an irrigation pathway therebetween and therealong to a distal end of the outer tubular sleeve and a distal end of the middle tubular member, the outer tubular sleeve adapted to connect a source of irrigation fluid to the irrigation pathway;
an inner shaft rotatably received within the middle tubular member, a distal end of the inner shaft forming a surgical bur configured to cut tissue or bone upon rotation thereof, the surgical bur extending distally beyond the distal end of the outer tubular sleeve; and
at least one fluid exit port defined proximate the distal end of the outer tubular sleeve at the end of the irrigation passageway configured to direct irrigation fluid in a narrow stream toward the surgical bur, wherein an annular space between each respective fluid exit port at the end of the irrigation passageway is sealed to prevent irrigation fluid from obscuring the surgeon's view of the surgical bur.

11. The surgical instrument according to claim 10, wherein the middle tubular member includes at least one channel defined within an outer peripheral surface thereof configured to carry irrigation fluid from the fluid source to a corresponding number of fluid exit ports defined within the outer tubular sleeve.

12. The surgical instrument according to claim 11, wherein the annular space between the distal end of the outer tubular sleeve and the distal end of the middle tubular member is sealed to prevent leakage of the irrigation fluid.

13. The surgical instrument according to claims 10, 11 or 12, wherein one fluid exit port is defined within the outer tubular sleeve and wherein the annular space between the distal end of the outer tubular sleeve and the distal end of the middle tubular member is sealed to prevent irrigation fluid from leaking from the annular space and obscuring the surgeon's view of the surgical bur.

14. The surgical instrument according to claims 10, 11, 12 or 13, wherein the inner shaft includes a proximal section and a distal section, at least one of the proximal or distal sections being made from a material enabling the respective section to bend allowing the inner shaft to bend in accordance with the shape of the outer tubular sleeve.

15. The surgical instrument according to claims 10, 11, 12, 13 or 14, wherein the inner shaft includes a proximal section and a distal section, at least one of the proximal or distal sections including a geometry enabling the respective section to bend allowing the inner shaft to bend in accordance with the shape of the outer tubular sleeve.

16. The surgical instrument according to claim 15, wherein the geometry of the inner shaft allowing it to bend includes segmenting.

17. The surgical instrument according to claims 15 or 16, wherein the inner shaft is segmented via at least one of laser cutting, die cutting, plasma cutting, flame cutting, drilling, metal turning, metal shape cutting, or water jet cutting.
